# EUROPEAN PATENT SPECIFICATION

(11) **EP 2 117 530 B1**
(45) Date of publication and mention of the grant of the patent: **09.01.2013**
(21) Application number: 08709349.8
(22) Date of filing: 07.02.2008
(51) Int. Cl.: A61K 31/7004, A61K 31/565, A61P 35/00

(54) **COMPOSITION COMPRISING 2DG AND STX140 FOR THE TREATMENT OF CANCER**
ZUSAMMENSETZUNG MIT 2DG AND STX140 ZUR BEHANDLUNG VON KREBS
COMPOSITION COMPRENANT 2DG AND STX140 POUR LE TRAITEMENT DU CANCER

(30) Priority: 08.02.2007 GB 0702446
(43) Date of publication of application: 18.11.2009
(73) Proprietor: Sterix Limited, Slough, Berkshire SL1 3XE (GB)
(72) Inventor: TAGG, Sarah, Louise, Claire, Berkshire SL1 3XE (GB); FOSTER, Paul, Alexander, Berkshire SL1 3XE (GB); NEWMAN, Simon, Paul, Berkshire SL1 3XE (GB); REED, Michael, John, Berkshire SL1 3XE (GB); PUROHIT, Atul, Berkshire SL1 3XE (GB); POTTER, Barry, Victor, Lloyd, Berkshire SL1 3XE (GB)
(74) Representative: Alcock, David
(86) International application number: PCT/GB2008/000448
(87) International publication number: WO 2008/096155

(56) References cited:
- WO-A-02/098511
- WO-A-2006/124573
- US-A1- 2003 203 855
- AFT RL, ZHANG FW, GIUS D: "Evaluation of 2-deoxy-D-glucose as a chemotherapeutic agent: mechanism of cell death" BRITISCH JOURNAL OF CANCER, vol. 87, 2002, pages 805-812, XP002477200
- LEESE M P ET AL: "2-Substituted Estrandiol Bis-sulfamates, Multitargeted Antitumor Agents: Synthesis, In Vitro SAR, Protein Crystallography, and In Vivo Activity" J MED CHEM, vol. 49, 2006, pages 7683-7696, XP002477239

## Description

### FIELD OF INVENTION

The present invention relates to a composition comprising 2-deoty-D-glucose and STX140.

In particular the present invention relates to the composition and to the sequential use of the components of the composition. The present invention also relates to the use of the composition in therapy applications.

### BACKGROUND TO THE INVENTION

In 2004, breast and prostate cancer were the third and fourth most common form of cancer, respectively, in Europe (Boyle & Ferlay, 2005). Current treatments for advanced hormone-independent cancer are limited, with resistance and toxicity being common problems with many therapies (Gottesman et al., 2002). There is therefore, a requirement to develop more efficacious therapeutic interventions for treating cancer. Solid tumours are particularly difficult to treat because most conventional chemotherapeutic drugs and radiation therapy only target the rapidly growing peripheral cells, leaving the slower growing tumour cells in the core to survive (Brown & Giaccia, 1998).

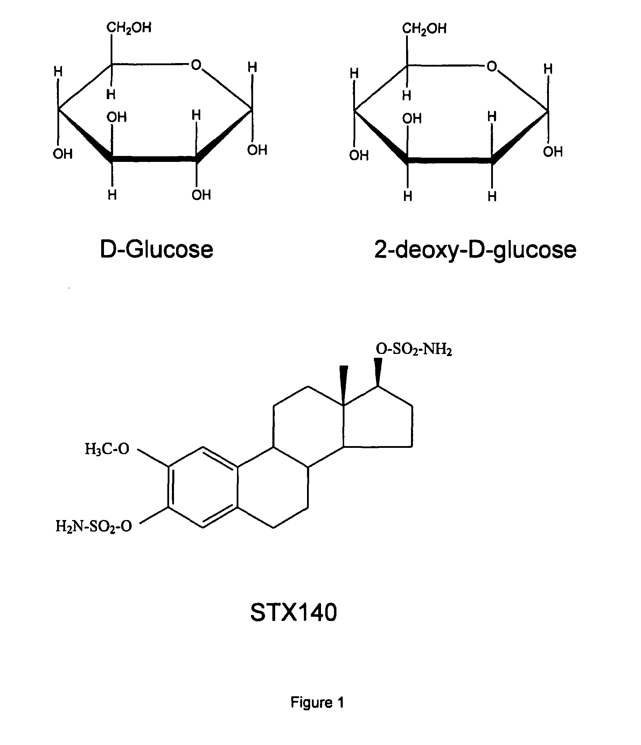

Normal, healthy cells undergo aerobic respiration to produce ATP, however, Warburg first demonstrated that even in the presence of oxygen, cancer cells create ATP anaerobically, relying on glucose for glycolysis (Warburg). Hence, inhibiting glycolysis could specifically target cancer cells; the effects on healthy normal cells should be minimal. Blocking glycolysis is possible by using 2-deoxy-D-glucose (2DG), which is structurally related to glucose and therefore, should not be toxic to healthy cells. The glucose molecule has a hydroxy group at its second carbon, however if this is changed to hydrogen, 2-deoxy-D-glucose (2DG) is formed (see Fig. 1). 2DG blocks glycolysis because when phosphorylated it cannot be converted to fructose-6-phosphate by phosphoglucose isomerase (Nirenberg et al., 1958), in contrast to glucose. In addition, 2DG competes with glucose for uptake by the hexose (GLUT) transporters (Kalir et al., 2002; Noguchi et al., 1999; Rudlowski et al., 2003).

2DG was shown to inhibit growth of adramycin-resistant MCF-7 cells which exhibit an enhanced rate of glycolysis (Kaplan et al., 1990). Several groups have shown 2DG to be an effective anti-tumour compound *in vitro* and *in vivo* (Aft et al., 2002; Gupta et al., 2005; Lampidis et al., 2006; Liu et al., 2001; Maschek et ai., 2004). However, very few clinical studies have tested 2DG as a single agent therapy and the results of such studies have been disappointing (Landau BR *et al.,* 1958, Kaplin O *et al.,* 1990). *In vivo* studies have provided strong evidence that using 2DG in combination with existing anti-cancer agents, such as adriamycin or paclitaxel may enhance efficacy (Maschek G *et al.,* 2004). For example, 2DG is currently being used in a Phase 1 trial in combination with the microtubule disruptor Docetaxel in patients with solid tumours (Raez et al., 2005). The microtubule disruptor Docetaxel would attack the rapidly growing cells and the inner core, reliant on glycolysis, could be targeted with 2DG. However, taxanes are toxic, not orally bioavailable and tumours often develop resistance to the taxanes (Polizzi et al, 1999).

### SUMMARY ASPECTS OF THE PRESENT INVENTION

The present invention is based on the surprising finding that action against tumours of a combination of a specific glycolytic inhibitor (ie.ZDF) and a specific compound (ie. STX140) is improved compared to the action of the materials alone or compared to what would be expected from the combination.

### DETAILED ASPECTS OF THE PRESENT INVENTION

According to one aspect of the present invention, there is provided claim 1.

According to one aspect of the present invention, there is provided claim 2.

According to one aspect of the present invention, there is provided claim 3.

According to one aspect of the present invention, there is provided claim 4.

According to one aspect of the present invention, there is provided claim 5.

According to one aspect of the present invention, there is provided claim 7.

According to one aspect of the present invention, there is provided claim 8.

For ease of reference, these and further aspects of the present invention are now discussed under appropriate section headings. However, the teachings under each section are not necessarily limited to each particular section.

### SOME ADVANTAGES

One key advantage of the present invention is that the compositions of the present invention can prevent and/or inhibit tumour angiogenesis.

One key advantage of the present invention is that the compositions of the present invention can modulate cell cycling.

One key advantage of the present invention is that the compositions of the present invention can modulate apoptosis.

One key advantage of the present invention is that the compositions of the present invention can modulate cell growth.

One key advantage of the present invention is that the compositions of the present invention can prevent and/or suppress glucose uptake by a tumour.

One key advantage of the present invention is that the compositions of the present invention can disrupt microtubules.

One key advantage of the present invention is that the compositions of the present invention can induce apoptosis.

The present invention is based on the surprising finding that the compositions provide an effective treatment of cancer.

Another advantage of the compositions of the present invention is that they may be potent in vivo.

Some of the compositions of the present invention are also advantageous in that they may be orally active.

The compositions of the present invention may useful for the prevention and/or treatment of cancer, such as breast cancer, as well as (or in the alternative) non-malignant conditions, such as the prevention and/or treatment of inflammatory conditions - such as conditions associated with any one or more of: autoimmunity, including for example, rheumatoid arthritis, type I and II diabetes, systemic lupus erythematosus, multiple sclerosis, myasthenia gravis, thyroiditis, vasculitis, endometriosis, ulcerative colitis and Crohn's disease, skin disorders e.g. acne, psoriasis and contact dermatitis; graft versus host disease; eczema; asthma and organ rejection following transplantation. In one aspect the compositions of the present invention may useful for the prevention and/or treatment of endometriosis. The compounds of the present invention are useful particularly when pharmaceuticals may need to be administered from an early age.

In one embodiment, the compounds of the present invention are useful for the treatment of breast cancer.

In one embodiment, the compounds of the present invention are useful for the treatment of prostate cancer.

In one embodiment, the compounds of the present invention are useful for the treatment of ovarian cancer.

Thus, some of the compounds of the present invention are also believed to have therapeutic uses other than for the treatment of endocrine-dependent cancers, such as the treatment of autoimmune diseases.

We have studied combinations of 2-deoxy-D-glycose [2DG] and the compound STX140 in MCF-7 and LNCaP cells *in vitro.* A MCF-7 (ER +ve) breast xenograft model was used to examine the potency of the composition on the growth of solid tumours. We have found that the present composition disrupt the rapidly dividing aerobic cells and offer a method of targeting both the hypoxic and aerobic cells in tumours.

We have also studied combinations of 2-deoxy-D-glucose [2DG] and the compound STX140 in MCF-7 and LNCaP cells *in vivo*. We have found that the present composition significantly reduced tumour growth. We have found that the present composition significantly reduced tumour growth. Moreover, use of the present composition may provide for lower doses to be used of compounds comprising a ring system substituted with at least one of a sulphamate group.

For ease of reference, these and further aspects of the present invention are now discussed under appropriate section headings. However, the teachings under each section are not necessarily limited to each particular section.

### PREFERABLE ASPECTS

The compound used in the present invention is

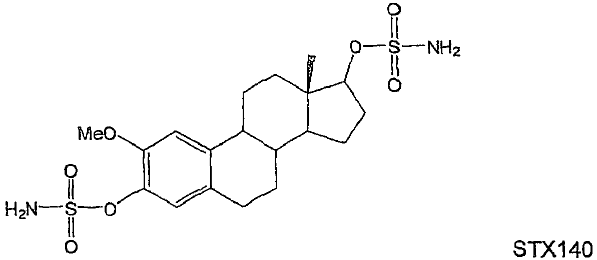

Distinct from Docetaxel, 2-Methoxyestradiol-3,17-O,O-*bis*-sulfamate (STX140) is a microtubule disruptor (Raobaikady 2005) which is orally bioavailable (Ireson 2004), not a substrate for p-glycoprotein (Suzuki, 2003) and can be dosed daily *in vivo* (Foster, submitted; Ireson 2004). The microtubule disruption leads to cell cycle arrest and subsequent apoptosis in both tumour and endothelial cells and inhibits *in vitro* angiogenesis (Newman et al., 2004) and *in vivo* angiogenesis (Foster, submitted; and Chander submitted).

STX140, was developed from the original steroid sulfatase (STS) inhibitor EMATE in order to overcome estrogenicity problems (Purohit et al, 1995- Biochemistry). The new class of A-ring modified anti-cancer compounds not only inhibited STS but were potent inhibitors of cell proliferation *in vitro* and in NMU induced tumours *in vivo* (Purohit et al, 2000, Int J Cancer).

The structurally related compound 2-MeOE2 is a well studied compound with known anti-cancer properties, and can inhibit cell proliferation and angiogenesis via a mechanism independent of hormone receptors (Fotsis et al., 1994). However, the clinical success of 2-MeOE2 has been limited as it has very poor oral bioavailability and is rapidly metabolised. In contrast to 2-MeOE2, STX140 avoids metabolism by 17βHSB due to its sulfate moiety at the C-17 position (Newman et al., 2006). STX140 has previously been shown to inhibit the proliferation of prostate LNCaP (androgen, AR +ve) cells (Day et al., 2003) and breast ER +ve MCF-7 cells (Suzuki et al., 2003b). STX140 inhibits doxorubicin and mitoxantrone resistant MCF-7 breast cancer proliferation (Suzuki et al., 2003a). The inhibition of *in vivo* angiogenesis by STX140 will increase hypoxia and make the tumour more reliant on glycolysis, thus sensitising the tumour to 2DG.

### COMPOSITION

As described above according to one aspect of the present invention, there is provided a pharmaceutical composition comprising a composition as defined herein, and a pharmaceutically acceptable carrier, diluent, excipient or adjuvant.

### THERAPY

The composition of the present invention may be used as therapeutic agents - i.e. in therapy applications.

The term "therapy" includes curative effects, alleviation effects, and prophylactic effects.

### PHARMACEUTICAL COMPOSITIONS

In one aspect, the present invention provides a pharmaceutical composition, which comprises a composition according to the present invention and a pharmaceutically acceptable carrier, diluent or excipient (including combinations thereof).

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

### COMBINATION PHARMACEUTICAL

In one instance of the present disclosure,

The composition of the present invention may be used in combination with one or more other active agents, such as one or more other pharmaceutically active agents.

By way of example, the composition of the present invention may be used in combination with other STS inhibitors and/or other inhibitors such as an aromatase inhibitor (such as for example, 4-hydroxyandrostenedione (4-OHA)) and/or steroids - such as the naturally occurring neurosteroids dehydroepiandrosterone sulfate (DHEAS) and pregnenolone sulfate (PS) and/or other structurally similar organic compounds.

In addition, or in the alternative, the composition of the present invention may be used in combination with a biological response modifier.

The term biological response modifier ("BRM") includes cytokines, immune modulators, growth factors, haematopoiesis regulating factors, colony stimulating factors, chemotactic, haemolytic and thrombolytic factors, cell surface receptors, ligands, leukocyte adhesion molecules, monoclonal antibodies, preventative and therapeutic vaccines, hormones, extracellular matrix components, fibronectin, etc. For some applications, preferably, the biological response modifier is a cytokine. Examples of cytokines include: interleukins (IL) - such as IL-1, IL-2, IL-3, IL-4, IL-5, IL-6, IL-7, IL-8. IL-9, IL-10, IL-11, IL-12, IL-19; Tumour Necrosis Factor (TNF) - such as TNF-α; Interferon alpha, beta and gamma; TGF-β. For some applications, preferably the cytokine is tumour necrosis factor (TNF). For some applications, the TNF may be any type of TNF - such as TNF-α, TNF-β, including derivatives or mixtures thereof. More preferably the cytokine is TNF-α. Teachings on TNF may be found in the art - such as WO-A-98/08870 and WO-A-98/13348.

### ADMINISTRATION

Typically, a physician will determine the actual dosage which will be most suitable for an individual subject and it will vary with the age, weight and response of the particular patient. The dosages below are exemplary of the average case. There can, of course, be individual instances where higher or lower dosage ranges are merited.

The compositions of the present invention may be administered by direct injection. The composition may be formulated for parenteral, mucosal, intramuscular, intravenous, subcutaneous, intraocular or transdermal administration. Depending upon the need, the agent may be administered at a dose of from 0.01 to 30 mg/kg body weight, such as from 0.1 to 10 mg/kg, more preferably from 0.1 to 1 mg/kg body weight.

By way of further example, the agents of the present invention may be administered in accordance with a regimen of 1 to 4 times per day, preferably once or twice per day. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

Aside from the typical modes of delivery - indicated above - the term "administered" also includes delivery by techniques such as lipid mediated transfection, liposomes, immunoliposomes, lipofectin, cationic facial amphiphiles (CFAs) and combinations thereof. The routes for such delivery mechanisms include but are not limited to mucosal, nasal, oral, parenteral, gastrointestinal, topical, or sublingual routes.

The term "administered" includes but is not limited to delivery by a mucosal route, for example, as a nasal spray or aerosol for inhalation or as an ingestable solution; a parenteral route where delivery is by an injectable form, such as, for example, an intravenous, intramuscular or subcutaneous route.

Thus, for pharmaceutical administration, the STS inhibitors of the present invention can be formulated in any suitable manner utilising conventional pharmaceutical formulating techniques and pharmaceutical carriers, adjuvants, excipients, diluents etc. and usually for parenteral administration. Approximate effective dose rates may be in the range from 1 to 1000 mg/day, such as from 10 to 900 mg/day or even from 100 to 800 mg/day depending on the individual activities of the compounds in question and for a patient of average (70Kg) bodyweight. More usual dosage rates for the preferred and more active compounds will be in the range 200 to 800 mg/day, more preferably, 200 to 500 mg/day, most preferably from 200 to 250 mg/day. They may be given in single dose regimes, split dose regimes and/or in multiple dose regimes lasting over several days. For oral administration they may be formulated in tablets, capsules, solution or suspension containing from 100 to 500 mg of compound per unit dose. Alternatively and preferably the compounds will be formulated for parenteral administration in a suitable parenterally administrable carrier and providing single daily dosage rates in the range 200 to 800 mg, preferably 200 to 500, more preferably 200 to 250 mg. Such effective daily doses will, however, vary depending on inherent activity of the active ingredient and on the bodyweight of the patient, such variations being within the skill and judgement of the physician.

### CELL CYCLING

The compounds of the present invention may be useful in the method of treatment of a cell cycling disorder.

It will be appreciated that cell cycling is an extremely important cell process. Deviations from normal cell cycling can result in a number of medical disorders. Increased and/or unrestricted cell cycling may result in cancer. Reduced cell cycling may result in degenerative conditions. Use of the compound of the present invention may provide a means to treat such disorders and conditions.

Thus, the compound of the present invention may be suitable for use in the treatment of cell cycling disorders such as cancers, including hormone dependent and hormone independent cancers.

In addition, the composition of the present invention may be suitable for the treatment of cancers such as breast cancer, ovarian cancer, endometrial cancer, sarcomas, melanomas, prostate cancer, pancreatic cancer etc. and other solid tumours.

For some applications, cell cycling is inhibited and/or prevented and/or arrested, preferably wherein cell cycling is prevented and/or arrested. In one aspect cell cycling may be inhibited and/or prevented and/or arrested in the G₂/M phase. In one aspect cell cycling may be irreversibly prevented and/or inhibited and/or arrested, preferably wherein cell cycling is irreversibly prevented and/or arrested.

By the term "irreversibly prevented and/or inhibited and/or arrested" it is meant after application of a compound of the present invention, on removal of the compound the effects of the compound, namely prevention and/or inhibition and/or arrest of cell cycling, are still observable. More particularly by the term "irreversibly prevented and/or inhibited and/or arrested" it is meant that when assayed in accordance with the cell cycling assay protocol presented herein, cells treated with a compound of interest show less growth after Stage 2 of the protocol I than control cells. Details on this protocol are presented below.

Thus, the present invention provides compounds which: cause inhibition of growth of oestrogen receptor positive (ER+) and ER negative (ER-) breast cancer cells in *vitro* by preventing and/or inhibiting and/or arresting cell cycling; and/or cause regression of nitroso-methyl urea (NMU)-induced mammary tumours in intact animals (i.e. not ovariectomised), and/or prevent and/or inhibit and/or arrest cell cycling in cancer cells; and/or act in *vivo* by preventing and/or inhibiting and/or arresting cell cycling and/or act as a cell cycling agonist.

### CELL CYCLING ASSAY

### Procedure

### Stage 1

MCF-7 breast cancer cells are seeded into multi-well culture plates at a density of 105 cells/well. Cells were allowed to attach and grown until about 30% confluent when they are treated as follows:
Control - no treatment
Compound of Interest (COI) 20µM

Cells are grown for 6 days in growth medium containing the COI with changes of medium/COI every 3 days. At the end of this period cell numbers were counted using a Coulter cell counter.

### Stage 2

After treatment of cells for a 6-day period with the COI cells are re-seeded at a density of 10⁴ cells/well. No further treatments are added. Cells are allowed to continue to grow for a further 6 days in the presence of growth medium. At the end of this period cell numbers are again counted.

### CANCER

As indicated, the composition of the present invention may be useful in the treatment of a cell cycling disorder. A particular cell cycling disorder is cancer.

We believe that the composition of the present invention provides a means for the treatment of cancers and, especially, breast cancer.

In addition or in the alternative the composition of the present invention may be useful in the blocking the growth of cancers including leukaemias and solid tumours such as breast, endometrium, prostate, ovary and pancreatic tumours.

In one instance of the present disclosure, the composition is believed to might be useful for :

### - THERAPY CONCERNING OESTROGEN

We believe that some of the composition of the present invention may be useful in the control of oestrogen levels in the body - in particular in females. Thus, the compound may be useful as providing a means of fertility control - such as an oral contraceptive tablet, pill, solution or lozenge. Alternatively, the compound could be in the form of an implant or as a patch.

Thus, the composition of the present invention may be useful in treating hormonal conditions associated with oestrogen.

In addition or in the alternative the composition of the present invention may be useful in treating hormonal conditions in addition to those associated with oestrogen. Hence, the composition of the present invention may also be capable of affecting hormonal activity and may also be capable of affecting an immune response.

### NEURODEGENERATIVE DISEASES

We believe that the composition of the present invention may be useful in the treatment of neurodegenerative diseases, and similar conditions.

By way of example, it is believed that the present composition may be useful in the enhancing the memory function of patients suffering from illnesses such as amnesia, head injuries, Alzheimer's disease, epileptic dementia, presenile dementia, post traumatic dementia, senile dementia, vascular dementia and post-stroke dementia or individuals otherwise seeking memory enhancement.

### - TH1

We believe that the composition of the present invention may be useful in TH1 implications.

### INFLAMATORY CONDITIONS

We believe that the compositions of the present invention may be useful in treating inflammatory conditions - such as conditions associated with any one or more of: autoimmunity, including for example, rheumatoid arthritis, type I and II diabetes, systemic lupus erythematosus, multiple sclerosis, myasthenia gravis, thyroiditis, vasculitis, ulcerative colitis and Crohn's disease, skin disorders e.g. psoriasis and contact dermatitis; graft versus host disease; eczema; asthma and organ rejection following transplantation.

The composition of the present invention may be useful in the manufacture of a medicament for revealing an endogenous glucocorticoid-like effect.

### OTHER THERAPIES

It is also to be understood that the composition of the present invention may have other important medical implications.

For example, the composition of the present invention may be useful in the treatment of the disorders listed in WO-A-99/52890 - *viz:*

In addition, or in the alternative, the composition of the present invention may be useful in the treatment of the disorders listed in WO-A-98/05635. For ease of reference, part of that list is now provided: cancer, inflammation or inflammatory disease, dermatological disorders, fever, cardiovascular effects, haemorrhage, coagulation and acute phase response, cachexia, anorexia, acute infection, HIV infection, shock states, graft-versus-host reactions, autoimmune disease, reperfusion injury, meningitis, migraine and aspirin-dependent anti-thrombosis; tumour growth, invasion and spread, angiogenesis, metastases, malignant, ascites and malignant pleural effusion; cerebral ischaemia, ischaemic heart disease, osteoarthritis, rheumatoid arthritis, osteoporosis, asthma, multiple sclerosis, neurodegeneration, Alzheimer's disease, atherosclerosis, stroke, vasculitis, Crohn's disease and ulcerative colitis; periodontitis, gingivitis; psoriasis, atopic dermatitis, chronic ulcers, epidermolysis bullosa; corneal ulceration, retinopathy and surgical wound healing; rhinitis, allergic conjunctivitis, eczema, anaphylaxis; restenosis, congestive heart failure, endometriosis, atherosclerosis or endosclerosis.

In addition, or in the alternative, the composition of the present invention may be useful in the treatment of disorders listed in WO-A-98/07859. For ease of reference, part of that list is now provided: cytokine and cell proliferation/differentiation activity; immunosuppressant or immunostimulant activity (e.g. for treating immune deficiency, including infection with human immune deficiency virus; regulation of lymphocyte growth; treating cancer and many autoimmune diseases, and to prevent transplant rejection or induce tumour immunity); regulation of haematopoiesis, e.g. treatment of myeloid or lymphoid diseases; promoting growth of bone, cartilage, tendon, ligament and nerve tissue, e.g. for healing wounds, treatment of burns, ulcers and periodontal disease and neurodegeneration; inhibition or activation of follicle-stimulating hormone (modulation of fertility); chemotactic/chemokinetic activity (e.g. for mobilising specific cell types to sites of injury or infection); haemostatic and thrombolytic activity (e.g. for treating haemophilia and stroke); antiinflammatory activity (for treating e.g. septic shock or Crohn's disease); as antimicrobials; modulators of e.g. metabolism or behaviour; as analgesics; treating specific deficiency disorders; in treatment of e.g. psoriasis, in human or veterinary medicine.

In addition, or in the alternative, the composition of the present invention may be useful in the treatment of disorders listed in WO-A-98/09985. For ease of reference, part of that list is now provided: macrophage inhibitory and/or T cell inhibitory activity and thus, antiinflammatory activity; anti-immune activity, i.e. inhibitory effects against a cellular and/or humoral immune response, including a response not associated with inflammation; inhibit the ability of macrophages and T cells to adhere to extracellular matrix components and fibronectin, as well as up-regulated fas receptor expression in T cells; inhibit unwanted immune reaction and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal trauma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery, bone marrow transplantation or other transplantation complications and/or side effects, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

In addition, or in the alternative, the composition of the present invention may be useful in the treatment of the disorders listed selected from endometriosis, uterus fibromyoma, induction of mono-ovulation (in polycystic ovarian disease [PCOD] patients), induction of multiple follicullar development in (ART patients), preterm labor/cervical incompetency and recurrent abortion.

### EXAMPLES

The present invention will now be described in further detail by way of example only with reference to the accompanying figures in which:-
Figure 1 shows: Structural difference between glucose and 2-deoxy-D-glucose. Glucose and 2DG differ at the second carbon. (B) Chemical structure of STX140.
Figure 2 shows: Effect of 2DG on the proliferation of LNCaP and MCF-7 cells. Cells were cultured in 96-well plates and treated with 2DG (0.005 mM - 50 mM) for 5 days when their effects on proliferation were measured using a microtiter plate assay.
Figure 3 shows: Effect of 2DG and STX140 on the proliferation of LNCaP (B) and MCF-7 (A) cells. Cells were cultured in 96-well plates, under normoxia or hypoxia, and treated with 2DG (8 mM) and STX140 (0.1 µM - 1 µM) for 3 days when their effects on proliferation were measured using a microtiter plate assay. A two-tailed Student's t-test assuming unequal variance was carried out, comparing values to normoxic untreated controls (black) or to 2DG (8mM) alone (grey). ns= not significant; *p<0.05; **p<0.01; ***p<0.001

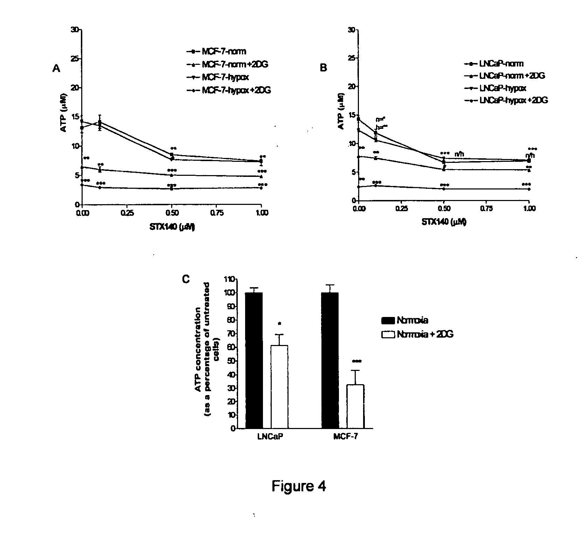

Figure 4 shows: Effect of 2DG and STX140 on the ATP levels of LNCaP (Figure 4B) and MCF-7 (Figure 4A) cells. Cells were cultured in 96-well plates, under normoxia or hypoxia, and treated with 2DG (8 mM) and STX140 (0.1 µM - 1 µM) for 3 days when their effects on ATP were measured using the microtiter, ATPlite plate assay. A two-tailed Student's t-test assuming unequal variance was carried out, comparing values to normoxic STX140 untreated controls (black) or to STX140 treated compared to normoxic untreated controls (grey). ns= not significant; **p*<0.05; ***p*<0.01; ****p*<0.001. Figure 4C shows percentage ATP change/live cell equivalent after 72h in LNCaP & MCF-7 cells in response to 2DG alone. A one-way ANOVA test was carried out. Comparing 2DG treated cells to untreated cells. LNCaP; n=3, MCF-7; n=2 **p*<0.05; ****p*<0.001}
Figure 5 shows: Morphology change in MCF-7 or LNCaP cells treated with compounds for 96 h, under normoxia.
Figure 6 shows: Growth of MCF-7 tumours in athymic nude mice. Growth of MCF-7 tumours (A) was inhibited by STX140 (5mg/kg) (p<0.05) and STX140 (5mg/kg) + 2DG (2g/kg) (p<0.01). There was no effect on mouse weight throughout the study (B) indicating a lack of compound toxicity. Points, mean percentage change in tumour volume (n=2-5 tumours); bars, SE, *p<0.05; **p<0.01 compared with control.
Figure 7 shows: Von Willebrand's factor staining of blood vessels. Administration of STX140 (5 mg/kg p.o.; daily) or STX140 (5 mg/kg p.o.; daily) + 2DG (2 g/kg i.p.; daily) caused a decrease in blood vessels relative to tumours taken from untreated animals. Magnification x 200.

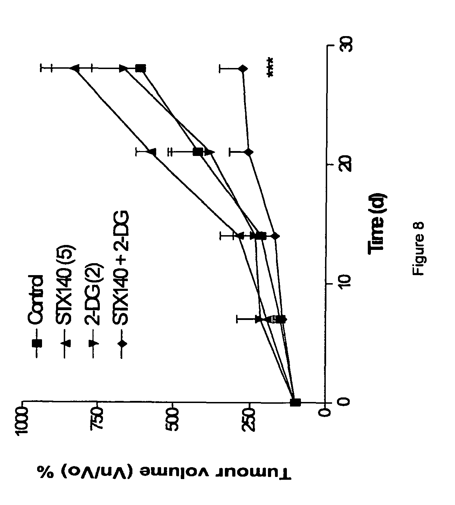

Figure 8 shows: Growth of LNCaP tumours in athymic nude mice. Growth of LNCaP tumours was inhibited by STX140 (5mg/kg) + 2DG (2g/kg) (p<0.001).

The present invention will now be described only by way of example. However, it is to be understood that the examples also present preferred compounds of the present invention, as well as preferred routes for making same and useful intermediates in the preparation of same.

### Materials and methods

### Compounds

2-MeOE2*bis*MATE (STX140) was synthesised at the Department of Pharmacy and Pharmacology, University of Bath (Leese et al., 2006). The compound displayed spectroscopic and analytical data in accordance with structure. 2-DEOXY_{-D-}GLUCOSE was obtained from Sigma (Irvine, England).

### Cell culture

MCF-7 and LNCaP cells were obtained from the American Tissue Culture Collection (ATCC). Cells were cultured in the following supplements, obtained from Sigma: RPMI 1640 with 10% foetal bovine serum, 1% L-glutamine, 1% MEM non-essential amino acids, 1% sodium bicarbonate solution, LNCaP cells also required 1% sodium pyruvate. Cells were maintained in an incubator at 37°C under 5% CO₂ and 95% air atmosphere.

### Proliferation assay

MCF-7 and LNCaP cells were seeded at 3000 cells/well or 5000 cells/well respectively, in 96 well plates. Compounds were added 4 hours after seeding of cells. Alamar blue 10 ml (Biosource, Nivelles, Belgium) was added and cell proliferation was measured 72 hrs later using a FluoStar Optima Plate reader.

### Hypoxia model

An Innova co-48 CO₂ humidified incubator (Newbrunswick Scientific, St. Albans, U.K.) was used to study the effects of 2DG and STX140 on cells under hypoxic conditions. Oxygen was maintained at 1 %, CO₂ 5% and temperature at 37°C.

### Measurement of ATP

MCF-7 and LNCaP cells were seeded at 3000 cells/well or 5000 cells/well respectively, in 96 well plates. Compounds were added 4 hours following seeding of cells. The ATPlite assay procedure was carried out according to manufacturer's instructions (Perkin Elmer, Beaconsfield, U.K.) and ATP measured 72 hrs later using a FluoStar Optima Plate reader.

### Photography

Cells were examined using a Zeiss inverted microscope fitted with a 20x and 40x Plan-Neofluor objective, and analysed through an Axiovision imaging system attached to the microscope.

### Immunoblotting

Immunoblotting was performed as previously described (Newman et al., 2004). The following antibodies were used: Anti-PAMPK;1: 1000 dilution (Millipore, Chanders Ford, U.K.), Anti-PBCL-2; 1:100 dilution (Cell Signaling, MA, U.S.A.), Anti-PP70S6K; 1:1000 dilution (Cell Signaling), Anti-phoshpo-SAPK/JNK; 1:1000 dilution (Cell Signaling), Anti-p21; 1:500 dilution (BD Biosciences, Oxford, U.K.) or anti-beta-actin; 1:3000 dilution (Abcam, Cambridge, U.K.) primary antibody, the wash was then repeated and the secondary antibodies added which were either anti-rabbit; 1:5000 dilution (Cell Signaling), anti-mouse; 1:5000 dilution (Cell Signaling) or anti-goat; 1: 5000 dilution (Abcam). After thoroughly washing the membrane it was developed using ECF substrate (Amersham Biosciences, Little Chalfont, U.K.) and analysed using a STORM Optical Scanner. Whole cell lysate was loaded onto the immunoblot, a Bradford assay was used to assess the protein content of the nuclear extracts to ensure equal protein loading in each lane.

### In vivo tumour xenograft model

### MCF-7 cell tumours

Intact, ovariectomized, athymic, female MF-1 nude mice (nu-/nu-) were purchased from Harlan (Bicester, Oxon, UK) at 5 weeks of age (-20-25g in weight). All experiments were carried out under conditions that complied with institutional guidelines. Animals were kept in a 12-hour light/12-hour dark cycle and given food and water *ad libitum.* Monolayers of MCF-7 cells were removed by trypsinization, and the resultant cell suspension was centrifuged for 5 min. at 1,000 x *g* and then resuspended in ice-cold matrigel (BD Biosciences). Five million MCF-7 cells were injected s.c. into the right flank of the animals. The tumours were allowed to grow for 2 weeks, when the tumours reached 100-150 mm³ in volume, mice were randomly divided into four treatment groups: 1) vehicle (10% tetrahydrofuran; 90% propylene glycol, 100ml oral) + saline i.p. 200 ml; 2) vehicle + 2DG (2g/kg) i.p. 200 ml; 3) STX140 (5mg/kg) 100ml oral + saline i.p. 200 ml; 4) STX140 (5mg/kg) 100ml oral + 2DG (2g/kg) i.p. 200 ml. All treatments were administered daily for 4 weeks, 2DG was given i.p. (100 ml) and STX140 or vehicle was given orally (200 ml). Throughout the study, mice were weighed and tumour measurements were taken on a weekly basis. Tumour volumes were calculated using the formla: length x width² /2. At the end of the study, tumours were collected for immunohistochemical analysis.

### LNCaP cell tumours

Athymic, male MF-1 nude mice (nu-/nu-) were purchased from Harlan (Bicester, Oxon, UK) at 5 weeks of age (-20-25g in weight). All experiments were carried out under conditions that complied with institutional guidelines. Animals were kept in a 12-hour light/12-hour dark cycle and given food and water *ad libitum.* Monolayers of LNCaP cells were removed by trypsinization, and the resultant cell suspension was centrifuged for 5 min. at 1,000 x *g* and then resuspended in ice-cold matrigel (BD Biosciences). Five million LNCaP cells were injected s.c. into the right flank of the animals. The tumours were allowed to grow for 3 weeks, when the tumours reached 100-150 mm³ in volume, mice were randomly divided into four treatment groups: 1) vehicle (10% tetrahydrofuran; 90% propylene glycol, 100µl p.o. + saline i.p. 200 µl; 2) vehicle p.o. + 2DG (2g/kg) i.p. 200 µl; 3) STX140 (5mg/kg) 100µl p.o. + saline i.p. 200 µl; 4) STX140 (5mg/kg) 100µl p.o. + 2DG (2g/kg) i.p. 200 µl. All treatments were administered 5 days per week for 4 weeks, 2DG was given i.p. (200 µl) and STX140 or vehicle was given orally (100 µl). Throughout the study, mice were weighed and tumour measurements were taken on a weekly basis. Tumour volumes were calculated using the formula: length x width² /2.

### Immunohistochemistry

von Willebrand's factor IHC were performed on paraffin embedded MDA-MB-231 tumor sections cut at 6 mm. After sectioning, rehydration and antigen retrieval steps, von Willebrand antibody (1:800, Abcam, Cambridge, United Kingdom) was applied to the section for 1 h at RT, followed by a goat polyclonal secondary antibody conjugated to FITC (30 min at RT). Sections were then mounted and viewed under a light or fluorescence microscope.

### Statistics

All experiments were carried out in triplicate and data presented are representative of one of three such experiments, unless indicated. All errors shown are the mean ± SE. Student's t test was used to assess the significance of the differences in cell proliferation for all experiments and ANOVA was used to look at the percentage change in ATP per live cell equivalent.

### Results

### Cell Proliferation assays: Effect of 2DG alone.

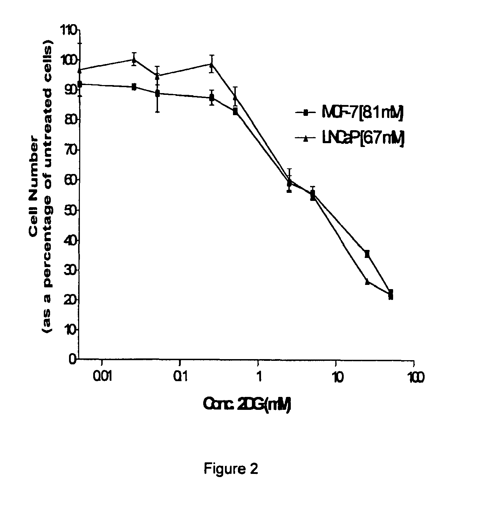

The ability of 2DG to inhibit the proliferation of LNCaP and MCF-7 cell lines was examined over a 5 day period under normoxic conditions (Fig. 2). The graph clearly shows a reduction in MCF-7 and LNCaP cell proliferation with increased 2DG concentration. The potency of 2DG was similar in both cell lines (MCF-7; IC₅₀: 8.1 mM and LNCaP; IC₅₀: 6.7 mM). The differences in IC₅₀ between MCF-7 and LNCaP cells were not significant. Several groups have carried out experiments using 2DG at concentrations between 1-24 mM (Aft et al., 2002; Kaplan et al., 1990; Lampidis et al., 2006; Zhu et al., 2005) and based on these findings, 8 mM 2DG was used throughout the study.

### Cell Proliferation assays: Effect of 2DG and STX140, alone and in combination.

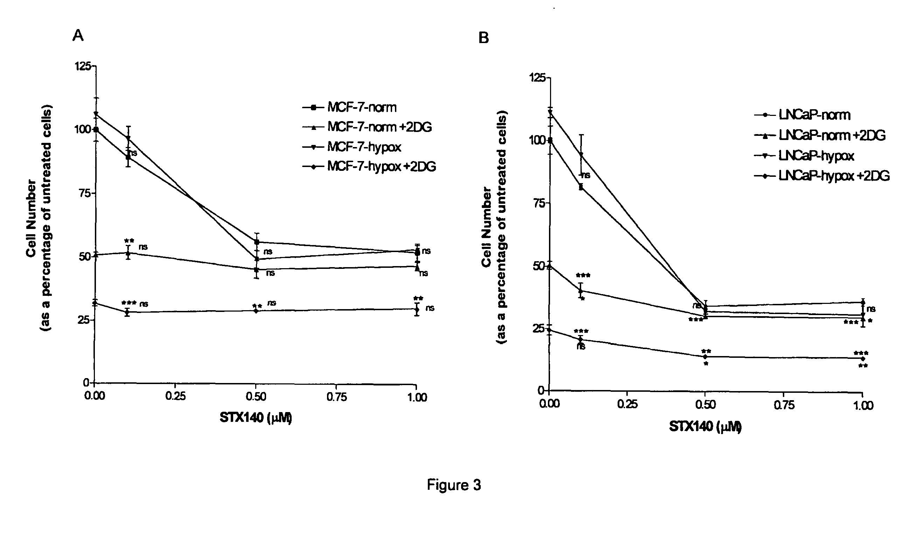

The growth inhibitory effects of 2DG and STX140, used alone and in combination were compared (Fig. 3). Two cell lines were utilised, under normoxic or hypoxic conditions. The growth inhibition was determined after 72 h. Compared to normoxic untreated controls, STX140 (0.5 µM) inhibited cell proliferation by 65 % in LNCaP cells (p<0.001, Figure 3b) both under normoxia and hypoxia and by 45 % and 48 % in MCF-7 cells (p<0.01, Figure 3a) under normoxia and hypoxia respectively. The IC₅₀ was calculated in LNCaP cells, both in normoxic (293 nM) and hypoxic (316 nM) conditions. These IC₅₀ values were not significantly different, and similar values have previously been calculated by Day and Newman (MCF-7: IC₅₀; 250 nM and LNCaP: IC₅₀; 260 nM) (Day et al., 2003; Newman et al., 2004). Under normoxic conditions, in both cell types, 2DG alone (8 mM) inhibited cell growth by 50% (LNCaP : p<0.01 and MCF-7: p<0.001). Hypoxic conditions further decreased cell growth to 70% (MCF-7: p<0.001) and 75% (LNCaP: p<0.001), Compared to STX140 at 0.5 µM, 2DG (8 mM) is significantly more effective at inhibiting tumour cell growth in both cell types (MCF-7: p<0.01 and LNCaP: p<0.05) under hypoxia. The ability of STX140 and 2DG combined to inhibit cell proliferation was studied in both cell types. In LNCaP cells the combination of 2DG and STX140 significantly decreased cell number, whether in normoxia or hypoxic conditions, compared to normoxic, untreated control. Under normoxic conditions 2DG (8 mM) reduces cell proliferation by 50%, this is increased to 60% (p<0.05) at 0.1 µM STX140, 68% (p<0.001) at 0.5 µM STX140 and 70% (p<0.001) at 1 µ STX140.

Under hypoxic conditions, 2DG alone reduces the proliferation of LNCaP cells by 75%, this inhibition is further decreased to 85% (p<0.05) with addition of STX140 (0.5 µ) down to 87% (p<0.01) with addition of STX140 (1 µ).

### Effect of 2DG and STX140 on ATP levels

The ATP levels of MCF-7 and LNCaP treated with 2DG and STX140 were studied (Fig. 4a and b). Cells were incubated under normoxic or hypoxic conditions and ATP measured after 72 h. The concentration of ATP drops significantly in both cell types after treatment with STX140 (p<0.01-p<0.001) compared to untreated normoxic controls. 2DG used alone decreases ATP in normoxia (p<0.01) and hypoxia (p<0.001) compared to untreated normoxic controls. 2DG and STX140 reduce ATP levels at all concentrations of STX140 under normoxia (p<0.05-0.001) and hypoxia shows the greatest reduction in ATP (p<0.001) compared to untreated normoxic controls. The effect of 2DG on ATP concentration was calculated per living MCF-7 or LNCaP cell (Fig. 4c). 2DG (8 mM) significantly decreases ATP in living cells; LNCaP (p<0.05) and MCF-7 (p<0.001) compared to untreated controls. Karczmar et al showed tumour ATP reduction caused by 2DG (Karczmar et al., 1992).

### Cell morphology

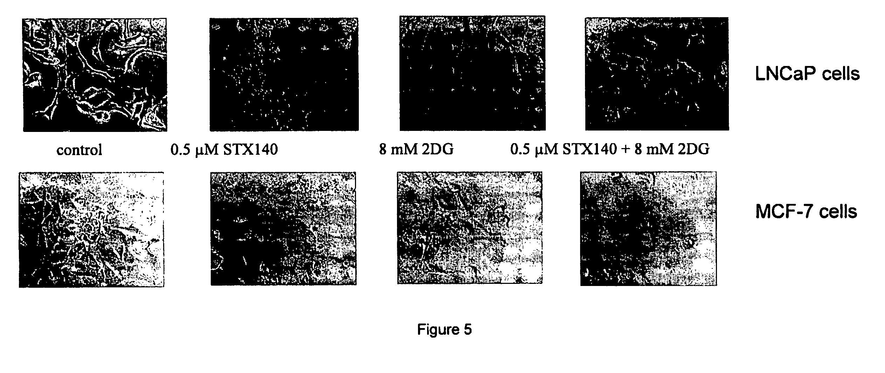

Images show LNCaP and MCF-7 cells treated with STX140 (0.5 µM) and 2DG 8 mM) alone or in combination (Fig. 5). Untreated cells look healthy compared to the shrivelled appearance of STX140 treated cells. When 2DG is added the cells appear to swell up, possibly due to 2DG being trapped in the cell and osmosis occurring. The cells look less healthy when the compounds are combined, with shrivelled, swollen apoptotic-looking cells.

### Effect of STX140 and 2DG in vivo

### MCF-7 cell tumours

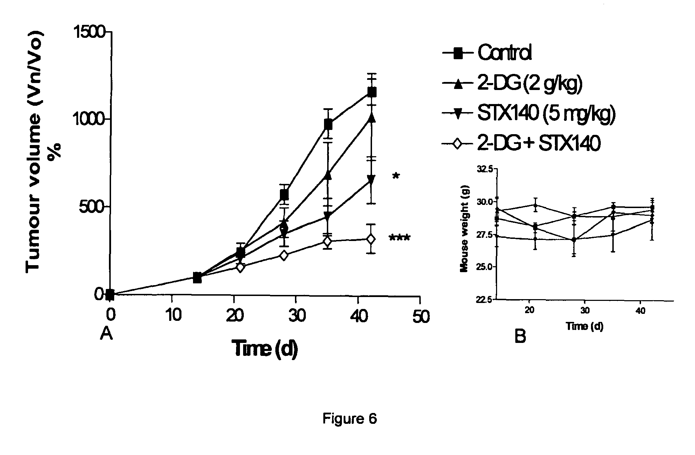

Following inoculation, MCF-7 cells developed into stable growing tumours, with a "take rate" of 80%. Once tumours had reached approximately 100-150 mm³ in volume, compound dosing commenced for 28 days. At the end of dosing growth of the MCF-7 tumours (Fig. 6a) was significantly inhibited by STX140 (5 mg/kg p.o.; daily) (p<0.05) compared to control. 2DG (2 glkg i.p.; daily) alone did not significantly inhibit tumour growth, compared to control. However, the combination of STX140 (5 mg/kg p.o.; daily) and 2DG (2 g/kg i.p.; daily) did significantly inhibit tumour proliferation (p<0.001). Figure 6b reveals the changes in the weights of mice throughout the study. No weight loss occurred, indicating that the animals tolerated the compounds without any toxicity.

### Expression of von Willebrand's factor

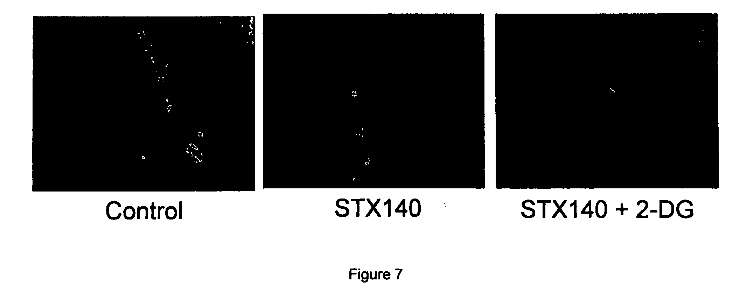

The endothelial specific marker, von Willebrand's factor, was used to assess vessel density in sections taken from MCF-7 xenografts taken from treated animals. Figure 7 shows both STX140 (5 mg/kg p.o.; daily) and STX140 (5 mg/kg p.o.; daily) combined with 2DG (2g/kg i.p.; daily) caused a significant reduction in the staining for endothelial cells. 2DG (2g/kg i.p.; daily) alone had no significant effect (data not shown).

### LNCaP cell tumours

Once tumours had reached approximately 100-150 mm³ in volume, compound dosing commenced for 5 days per week for 4 weeks. At the end of dosing growth of the LNCaP tumours was significantly inhibited by the combination of STX140 (5 mg/kg p.o.) and 2DG (2 g/kg i.p.) (p<0.001).

### Discussion

The aim of this study was to improve inhibition of tumour cell proliferation using the combination of an alkoxy/sulphamate substituted ring system compound and a glycolytic inhibitor *in vitro* and *in vivo.* Tumour cells, even in the presence of oxygen, continue to rely on glycolysis rather than oxidative phosphorylation (Warburg, 1956). Several oncogenes have been implicated in the Warburg effect; the AKT oncogene is associated with enhanced glucose uptake and aerobic glycolysis, independent of HIF-1 (Elstrom et al., 2004). AKT assembles glucose transporters to the cell surface to enhance glucose uptake and activate hexokinase It (HK2) to phosphorylate and trap intracellular glucose. AKT is able to enhance glycolytic flux without affecting mitochondrial oxidative phosphorylation, contributing to the Warburg effect. The MYC oncogene activates virtually all glycolytic enzyme genes, including those encoding HK2, enolase, and lactate dehydrogenase A (LDHA) (Kim & Dang, 2005). The inner core of tumour cells are particularly resistant to many of the anti-cancer agents which target rapidly dividing cells (Liu et al., 2001).

Anaerobically metabolizing, slow growing tumour cells demonstrate another form of multidrug resistance (MDR) in addition to the MDR mechanisms previously identified, for example the ABC cassette family of transporter proteins, P-GP and MRP (Hendrikse, 2000; Kuwano et al., 1999) and topoisomerase (Fortune & Osheroff, 2000; Gupta et al., 2005).

STX140 causes caspase-dependent apoptosis in CAL51 breast cancer cells and overcomes resistance to TRAIL by activating caspases (Wood et al., 2004). STX140 has been shown to be 6 times more potent an inhibitor of breast cancer cells compared to 2-MeOE2 (Raobaikady et al., 2003) and can inhibit proliferation of HUVEC cells 60 fold more effectively than 2-MeOE2 and was 10-13 times more active as an inhibitor of vessel formation in a novel co-culture model system (Newman et al., 2004). STX140 was also effective in cells resistant to mitoxantrone or doxorubicin (Suzuki et al., 2003a) and was also a very active anti-tumour agent *in vivo* (Ireson et al., 2004).

STX140 also has good STS inhibitory properties *in vitro* and *in vivo* (Raobaikady et al., 2003) which is beneficial for the treatment of hormone dependent breast cancers. STX140 shows enhanced oral availability and improved pharmacokinetic properties (Ireson et al., 2004). Ho *et al.,* showed that TNF-aand STX140 used together increased the potency of 2-substituted oestrogens as anti-angiogenic agents via synergistic induction of apoptosis in endothelial cells and had low cytotoxicity in fibroblasts (Ho et al., 2003). Fluorescent images of MDA-MB-231 cells treated with STX140 revealed disintegrated tubulin, condensed nuclei and microtubule damage. In addition, STX140 caused cell cycle arrest in MDA-MB-231 cells (Raobaikady et al., 2005). To summarise the anti-tumour affects of STX140 are caused by disruption of microtubules leading to cell cycle arrest and subsequent apoptosis. Furthermore, STX140 has potential *in vitro* and *in vivo* anti-angiogenic activity (Newman 2004, Chander 2007[submitted] and Foster et al., 2007 [submitted]).

The results from this study confirm that 2DG is a potent inhibitor of both LNCaP and MCF-7 cell proliferation, with increased potency under hypoxic conditions. Other groups have also showed 2DG to be very efficacious at inhibiting tumour cell growth (Lyon et al., 1988 and Kaplan et al., 1990).

In addition, Liu *et al* used two models to investigate the tumour cells dependency on glycolysis. The first represented osteosarcoma wild-type (wt) cells treated with agents which inhibit mitochondrial oxidative phosphorylation (Oxphos) by interacting with complexes I, III and V of the electron transport chain in different ways, inhibitors included Rhodamine (Rho 123), rotenone, antimycin A and oligomycin. All of these Oxphos inhibitors were found to hypersensitize wild-type cells to 2DG. Cells treated with Rho 123 also became hypersensitive to oxamate, an analogue of pyruvate which blocks the step of glycolysis that converts pyruvate to lactic acid. The second model is r^{□} cells which have lost their mitochondrial DNA and therefore cannot undergo Oxphos. These cells were 10 and 4.9 times more sensitive to 2DG than oxamate, respectively, than wt cells. Overall, Liu et al showed that the glycolytic inhibitors oxamate and 2DG could be used to specifically target the slow-growing cells of a tumour and thereby increase the efficacy of current chemotherapeutic and irradiation protocols designed to kill rapidly dividing cells. They also hypothesised that glycolytic inhibitors could be particularly useful in combination with anti-angiogenic agents which should make tumours more anaerobic and therefore, reliant on glycoloysis (Liu et al., 2001).

Aft *et al* studied the effects of 2DG on breast cancer cells *in vitro:* 2DG treatment of breast cancer cells resulted in cessation of cell growth in a dose-dependent manner. Cell death was induced by 2DG caused by apoptosis, with induction of caspase 3 activity and cleavage of poly (ADP-ribose) polymerase. GLUT 1 transporter was elevated and glucose uptake elevated compared to non-2DG treated breast cancer cells. It was concluded that 2DG causes death in human breast cancer cells by activation of the apoptotic pathway, they accelerate their own demise by initially expressing high levels of glucose transporter protein, which allowed increased uptake of 2DG and subsequent induction of cell death. Aft *et al* revealed good evidence to support the targeting of glucose metabolism as a site for chemotherapeutic intervention by agents such as 2DG (Aft et al., 2002).

The results of this study revealed how 2DG depletes ATP *in vitro.* Xu et al showed how inhibition of glycolysis severely depleted ATP in cancer cells, especially those cells with mitochondrial defects and led to rapid dephosphorylation of the glycolysis-apoptosis interacting molecule BAD at Ser¹¹², relocalization of BAX to mitochondria and massive cell death. 2DG was shown to utilise a novel strategy to overcome drug resistance associated with mitochondrial respiratory defect and hypoxia (Xu et al., 2005).

Zhu *et al* showed in MCF-7 cells that 2DG reduced cell growth and intracellular ATP in a dose- and time- dependent manner. 2DG increased levels of phosphorylated AMPK and Sirt-1 and reduced phosphorylation of Akt. This study supported the hypothesis that dietary energy restriction (DER) inhibits carcinogenesis, partly by limiting glucose availability and that energy metabolism is a target for the development of energy restriction mimetic agents for chemoprevention (Zhu et al., 2005). Recently, Lampidis et al created 2-halogenated D-glucose analogs for improved activity, compared to 2DG. 2-fluor-2-deoxy-D-glucose (2FG) was more potent than 2DG in killing hypoxic tumour cells and would therefore could be more clinically effective when combined with standeard chemotherapeutic protocols (Lampidis et al., 2006).

The results of this study revealed how there is significant improvement in cell proliferation inhibition when a glycolytic inhibitor (such as 2DG) is combined with a alkoxy/sulphamate substituted ring system compound (such as STX140) in LNCaP cells. The combination of compounds was not more effective than either agent alone in MCF-7 cells *in vitro.* The *in vitro* proliferation assay used in these studies does not reflect the *in vivo* situation; there is no inner core of slow growing tumour cells that would be potentially recalcitrant to STX140 but sensitive to 2DG. The true benefit of combining agents with 2DG may only become apparent using *in vivo* tumour models.

The combination of 2DG and STX140 is the most effective at inhibiting tumour xenograft growth compared to either agent alone, in vivo. The dose of STX140 used *in vivo* was only 5 mg/kg, it would be possible to increase the efficiency of STX140 by increasing the dose to 20 mg/kg as used by Foster *et al* (Foster et al., 2006[submitted]).

Several groups have also showed beneficial effect when chemotherapeutic agents are combined with 2DG, with the hope of targeting the aerobic rapidly dividing and anaerobic, slowly dividing cells: Maschek et al utilised 2DG in combination with Adriamycin (ADR) or Paclitaxel in nude mouse xenograft models of human osteosarcoma and non-small cell lung cancer. Nude mice were implanted with osterosarcoma cells and divided into four groups: 1) untreated, 2) ADR alone, 3) 2DG alone, 4) ADR + 2DG. Tumours were 50 mm³ or 300 mm³ in volume when treatment began. Starting with small or large tumours, the ADR + 2DG combinations resulted in significantly slower tumour growth (and therefore longer survival) then the control, 2DG or ADR treatments (p<0.0001). Similar beneficial effects of combination treatment were found with 2DG and paclitaxel in MV522 non-small lung cancer xenograft model. Their results provided a rationale for initiating clinical trials using glycolytic inhibitors in combination with chemotherapeutic agents to increase their therapeutic effectiveness (Maschek et al., 2004). In 2005, Gupta et al showed how 2DG enhances the efficacy of etoposide in Ehrlich ascites tumour-bearing mice. Analysis of cells obtained from ascitic fluid as well as solid tumours during follow up- revealed etoposide induced cell death was mainly due to apoptosis, which was enhanced further by 2DG. In addition, 2DG did not increase the toxicity of etoposide at 60mg/kg. Therefore, administration of 2DG can improve the local control of tumours without increasing normal tissue toxicity, so enhancing the therapeutic efficacy of topoisomerase inhibitor -based anti-cancer drugs like etoposide (Gupta et al., 2005).

The results of this study revealed how STX140 causes a decrease in von Willebrand's factor staining, thus supporting the anti-angiogenic potential of this compound observed *in vitro* (Newman et al., 2004). The combination of STX140 and 2DG was the most efficacious at inhibiting *in vivo* tumour growth, this supports the hypothesis that the present anti-angiogenic agents may potentiate the activity of 2DG due to an increase in hypoxia and greater reliance on glycolysis.

Raez et al have instigated a Phase 1 trial of 2DG and cocetaxel in patients with solid tumours and the drug combination was shown to be feasible and safe (Raez et al., 2005)

In conclusion, both STX140 and 2DG are very potent anti-cancer compounds *in vitro* and *in vivo.* There are clear advantages of using multi-targeting treatments, combining anti-angiogenic activity, microtubule disruption with 2DG to attack the whole of the tumour. The anti-angiogenic properties of STX140 results in less blood vessels, an increase in hypoxia and therefore more glycolysis, thus sensitizing the cell to 2DG. The microtubule disruption of STX140 will target rapidly dividing cells, inhibiting cell proliferation. Using multiple compounds reduces the likelihood of resistance so lower doses of microtubule disruptors can be used causing less off target effects. In the future it may be very beneficial to modify 2DG in order to make it more efficacious in cancer prevention.

### REFERENCES

1. Aft, R.L., Zhang, F.W. & Gius, D. (2002). Evaluation of 2-deoxy-D-glucose as a chemotherapeutic agent: mechanism of cell death. Br J Cancer, 87, 805-12.
2. Aloj, L., Caraco, C., Jagoda, E., Eckelman, W.C. & Neumann, R.D. (1999). Glut-1 and hexokinase expression: relationship with 2-fluoro-2-deoxy-D-glucose uptake in A431 and T47D cells in culture. Cancer Res, 59, 4709-14.
3. Arora, K.K., Parry, D.M. & Pedersen, P.L. (1992). Hexokinase receptors: preferential enzyme binding in normal cells to nonmitochondrial sites and in transformed cells to mitochondrial sites. J Bioenerg Biomembr, 24, 47-53.
4. Attalla, H., Westberg, J.A., Andersson, L.C., Aldercreutz, H. & Makela, T.P. (1998). 2-Methoxyestradiol-induced phoshphorylation of Bcl-2: Uncoupling from JNK/SAPK activationl. Biochem Biophys Res Commun, 247, 616-619.
5. Basu, A. & Haldar, S. (2003). Identification of a novel Bcl-xL phosphorylation site regulating the sensitivity of taxol- or 2-methoxyestradiol-induced apoptosis. FEBS Lett, 538, 41-47.
6. Birnbaum, M.J., Haspel, H.C. & Rosen, O.M. (1987). Transformation of rat fibroblasts by FSV rapidly increases glucose transporter gene transcription. Science, 235, 1495-8.
7. Boyle, P. & Ferlay, J. (2005). Cancer incidence and mortality in Europe, 2004. Annals of Oncology, 1-8.
8. Carew, J.S. & Huang, P. (2002). Mitochondrial defects in cancer. Mol Cancer, 1, 9.
9. Carew, J.S., Zhou, Y., Albitar, M., Carew, J.D., Keating, M.J. & Huang, P. (2003). Mitochondrial DNA mutations in primary leukemia cells after chemotherapy: clinical significance and therapeutic implications. Leukemia, 17, 1437-47.
10. Carothers, A.M., Hughes, S.A., Ortega, D. & Bertagnolli, M.M. (2002). 2-Methoxyestradiol induces p53-associated apoptosis of colorectoral cancer cells. Cancer Lett, 187, 77-86.
11. Chauhan, D., Catley, L., Hideshima, T. & al, e. (2002). 2-Methoxyestradiol overcomes drug resistance in multiple myeloma cells. Blood, 100, 2187-2194.
12. Cohen, P. (1997). The search for physiological substrates of MAP and SAP kinases in mammalian cells. Trends Cell Biol., 7, 353-361.
13. D'Amato, R.J., Lin, C.M., Flynn, E., Folman, J. & Hamel, E. (1994). 2-Methoxyestradiol, an endogenous mammalian metabolite, inhibits tubulin polymerization by interacting at the colchicine site. Proc Natl Acad Sci U S A, 91, 3964-3968.
14. Day, J.M., Newman, S.P., Comninos, A., Solomon, C., Purohit, A., Leese, M.P., Potter, B.V. & Reed, M.J. (2003). The effects of 2-substituted oestrogen sulphamates on the growth of prostate and ovarian cancer cells. J Steroid Biochem Mol Biol, 84, 317-25.
15. Dennis, P.B., Jaeschke, A., Saitoh, M., Fowler, B., Kozma, S.C. & Thomas, G. (2001). Mammalian TOR: a homeostatic ATP sensor. Science, 294, 1102-1105.
16. Elstrom, R.L., Bauer, D.E., Buzzai, M. & al, e. (2004). Akt stimualtes aerobic glycolysis in cancer cells. Cancer Res, 64, 3892-9.
17. Flier, J.S., Mueckler, M.M., Usher, P. & Lodish, H.F. (1987). Elevated levels of glucose transport and transporter messenger RNA are induced by ras or src oncogenes. Science, 235, 1492-5.
18. Fortune, J.M. & Osheroff, N. (2000). Topoisomerase II as a target for anticancer drugs: when enzymes stop being nice. Prog Nucleic Acid Res Mol Biol, 64, 221-53.
19. Foster, P.A., Newman, S.P., Chander, S.K., Stengal, C., Jhalli, R., Woo, L.L., Potter, B.V., Reed, M.J. & Purohit, A. (2006). In vivo Efficacy of STX213, A Second-Generation Steroid Sulfatase Inhibitor, for Hormone-Dependent Breast Cancer Therapy. Clin Cancer Res, 12, 5543-5549.
20. Fotsis, T., Zhang, Y., Pepper, M.S., Adlercreutz, H., Montesano, R., Nawroth, P.P. & Schweigerer, L. (1994). The endogenous oestrogen metabolite 2-methoxyoestradiol inhibits angiogenesis and suppresses tumour growth. Nature, 368, 237-9.
21. Fryer, L.G., Foufelle, F., Barnes, K., Baldwin, S.A., Woods, A. & Carling, D. (2002). Characterization of the role of the AMP-activated protein kinase in the stimulation of glucose transport in skeletal muscle cells. Biochem J, 363, 167-74.
22. Gallagher, B.M., Fowler, J.S., Gutterson, N.I., MacGregor, R.R., Wan, C.N. & Wolf, A.P. (1978). Metabolic trapping as a principle of oradiopharmaceutical design: some factors resposible for the biodistribution of [18F] 2-deoxy-2-fluoro-D-glucose. J Nucl Med, 19, 1154-61.
23. Geschwind, J.F., Georgiades, C.S., Ko, Y.H. & Pedersen, P.L. (2004). Recently elucidated energy catabolism pathways provide opportunities for novel treatments in hepatocellular carcinoma. Expert Rev Anticancer Ther, 4, 449-57.
24. Godov, A., Ulloa, V., Rodriquez, F., Reinicke, K., Yanez, A.J., Garcia Mde, L., Carrasco, M., Barberis, S., Castro, T., Martinez, F., Koch, X., Vera, J.C., Poblete, M.T., Figueroa, C.D., Peruzzo, B., Perez, F. & Nualart, F. (2006). Differential subcellular distribution of glucose transporters GLUT1-6 and GLUT9 in human cancer: untrastructural localization of GLUT1 and GLUT5 in breast tumor tissues. Journal of Cell Physiology, 207, 614-27.
25. Goldman, N.A., Katz, E.B., Glenn, A.S., Weldon, R.H., Jones, J.G., Lynch, U., Fezzari, M.J., Runowicz, C.D., Goldberg, G.L. & Charron, M.J. (2006). GLUT1 and GLUT8 in endometrium and endometrial adenocarcinoma. Mod Pathol.
26. Gottesman, M.M., Fojo, T. & Bates, S.E. (2002). Multidrug resistance in cancer: Role of ATP dependent transporters. Nature, 2, 48-58.
27. Gupta, S., Mathur, R. & Dwarakanath, B.S. (2005). The Glycolytic inhibitor 2-Deoxy-D-Glucose Enhances the Efficacy of Etoposide in Ehrlich Ascites Tumour-Bearing Mice. Cancer Biology & Therapy, 4, 87-94.
28. Hardie, D.G. & Carling, D. (1997). The AMP-activated protein kinase: fuel gauge of the mammalian cell. Eur J Biochem, 246, 259-273.
29. Hardie, D.G., Carling, D. & Carlson, M. (1998). The AMP-activated/SNF1 protein kinase subfamily: metabolic sensors of the eukaryotic cell? Annu. Rev. Biochem., 67, 821-855.
30. Hardie, D.G., Salt, LP., Hawley, S.A. & Davies, S.P. (1999). AMP-activated protein kinase: an ultrasensitive system for monitoring cellular energy charge. Biochem. J., 388, 717-722.
31. Hendrikse, N.H. (2000). Monitoring interactions at ATP-dependent drug efflux pumps. Curr Pharm Des., 6, 1653-68.
32. Ho, Y.T., Newman, S.P., Purohit, A., Leese, M.P., Potter, B.V. & Reed, M.J. (2003). The effects of 2-methoxy oestrogens and their sulphamoylated derivatives in conjunction with TNF-alpha on endothelial and fibroblast cell growth, morphology and apoptosis. Journal of Steroid Biochemistry & Molecular Biology, 86, 189-196.
33. Huang, P., Feng, L., Oldham, E.A., Keating, M.J. & Plunkett, W. (2000). Superoxide dismutase as a target for the selective killing of cancer cells.[see comment]. Nature, 407, 390-5.
34. Inoki, K., Zhu, T. & Guan, K.L. (2003). TSC2 Mediates Cellular Engergy REsponse to Control Cell Growth and Survival. Cell, 115, 577-590.
35. Ireson, C.R., Chander, S.K., Purohit, A., Perera, S., Newman, S.P., Parish, D., Leese, M.P., Smith, A.C., Potter, B.V. & Reed, M.J. (2004). Pharmacokinetics and efficacy of 2-methoxyoestradiol and 2-methoxyoestradiol-bis-sulphamate in vivo in rodents. British Journal of Cancer, 90, 932-7.
36. Jain, V.K., Kalia, V.K., Sharma, R., Maharajan, V. & Menon, M. (1985). Effects of 2-deoxy-D-glucose on glycolysis, proliferation kinetics and radiation response of human cancer cells. Int J Radiat Oncol Biol Phys, 11, 943-50.
37. Jordan, M.A. & Wilson, L. (1998). Microtubules and actin filaments: dynamic targets for cancer chemotherapy. Curr Opin Cell Biol, 10, 123-130.
38. Kalir, T., Wang, B.Y., Goldfischer, M., Haber, R.W. & al, e. (2002). Immunohistochemical staining of GLUT1 in benign, borderline, and malignant ovarian epithelia. Cancer, 94, 1078-1082.
39. Kaplan, O., Navon, G., Lyon, R.C., Faustino, P.J., Straka, E.J. & Cohen, J.S. (1990). Effects of 2-deoxyglucose on drug-sensitive and drug-resistant human breast cancer cells: toxicity and magnetic resonance spectroscopy studies of metabolism. Cancer Res, 50, 544-51.
40. Karczmar, G.S., Arbeit, J.M., Toy, B.J. & Weiner, M.W. (1992). Selective depletion of tumor ATP by 2-deoxyglucose and insulin, detected by [31]P magnetic resonance spectroscopy. Cancer Res, 52, 71-76.
41. Kemp, B.E., Mitchelhill, K.I., Stapleton, D., Michell, B.J., Chen, Z.-P. & Witters, L.A. (1999). Dealing with energy demand: the AMP-activated protein kinase. Trends Biochem. Sci., 24, 22-25.
42. Kern, K.A. & Norton, J.A. (1987). Inhibition of established rat fibrosarcoma growth by the glucose antagonist 2-deoxy-D-glucose. Surgery, 102, 380-5.
43. Kim, J.W. & Dang, C.V. (2005). Multifaceted roles of glycolytic enzymes. Trends Biochem. Sci., 30, 142-50.
44. Klauber, N., Parangi, S., Flynn, E., Hamel, E. & D'Amato, R.J. (1997). Inhibition of angiogenesis and breast cancer in mice by the microtubule inhibitors 2-methoxyestradiol and taxol. Cancer Res, 57, 81-86.
45. Koukourakis, M.I., Giatromanolaki, A., Polychronidis, A., Simopoulos, C., Gatter, K.C., Harris, A.L. & Sivridis, E. (2006). Endogenous markers of hypoxia/anaerobic metabolism and anemia in primary colorectal cancer. Cancer Sci, 97, 582-8.
46. Kumar, A.P., Garcia, G.E., Orsbom, J., Levin, V.A. & Slaga, T.J. (2003). 2-Methoxyestradiol interferes with. NF kappa B transcriptional activity in primitive neuroectodermal brain tumors: implications for management. Carcinogenesis, 24, 209-16.
47. Kuwano, M., Toh, S., Uchiumi, T., Takano, H., Kohno, K. & Wada, M. (1999). Multidrug resistance-associated protein subfamily transporters and drug resistance. Anticancer Drug Des., 14, 123-31.
48. Kyriakis, J.M. & Avruch, J. (1996). Sounding the alarm: protein kinase cascades activated by stress and inflammation. J Biol Chem, 271, 24313-6.
49. La Vallee, T.M., Zhan, X.H., Herbstritt, C.J., Kough, E.C., Green, S.J. & Pribluda, V.S. (2002). 2-Methoxyestradiol inhibitis proliferation and induces apoptosis independantly of estrogen receptors alpha and beta. Cancer Res, 62, 3691-3697.
50. Lampidis, T., Kurtoglu, M., Maher, J.C., Liu, H., Krishan, A., Sheft, V., Szymanski, S., Fokt, I., Rundnicki, W.R., Ginalski, K., Lesyng, B. & Priebe, W. (2006). Efficacy of 2-halogen substituted D-glucose analogs in blocking glycolysis and killing "hypoxic tumor cells". Cancer Chemother Pharmacol, 58, 725-734.
51. Laszlo, J., Humphreys, S.R. & Goldin, A. (1960). Effects of glucose analogues (2-deoxy-D-glucose, 2-deoxy-D-galactose) on experimental tumors. J Natl Cancer Inst, 24,267-81.
52. Landau BR, Laszlo J, Stengle J, Burk D. Certain metabolic and pharmacologic effects in cancer patients given infusions of 2-deoxy-D-glucose. J Natl Cancer Inst. 1958 Sep;21 (3):485-94.
53. LaVallee, T.M., Zhan, X.H., Johnson, M.S., Herbstritt, C.J., Swartz, G., Williams, M.S., Hembrough, W.A., Green, S.J. & Pribluda, V.S. (2003). 2-methoxyestradiol up-regulates death receptor 5 and induces apoptosis through activation of the extrinsic pathway. Cancer Research, 63, 468-75.
54. Lee, H.H., Dadgostar, H., Cheng, Q., Shu, J. & Cheng, G. (1999). NF-kappaB-mediated up-regulation of Bcl-x and Bfl-1/A1 is required for CD40 survival signaling in B lymphocytes. Proceedings of the National Academy of Sciences of the United States of America, 96, 9136-41.
55. Leese MP, Leblond B, Smith A, et al. 2-substituted estradiol bis-sulfamates, multitargeted antitumor agents: synthesis, in vitro SAR, protein crystallography, and in vivo activity. J Med Chem. 2006;49(26):7683-96.
56. Liu, H., Hu, Y.P., Savaraj, N., Priebe, W. & Lampidis, T.J. (2001). Hypersensitization of tumor cells to glycolytic inhibitors. Biochemistry, 40, 5542-5547.
57. Lyon, R.C., Cohen, J.S., Faustion, P.J., Megnin, F. & Myers, C.E. (1988). Glucose metabolism in drug-sensitive human breast cancer cells monitored by magnetic resonance spectroscopy. Cancer Res, 48, 870-7.
58. Mabjeesh, N.J., Escuin, D., La Vallee, T.M. & al, e. (2003). 2ME2 inhibits tumor growth and angiogenesis by disrupting microtubules and dysregulating HIF. Cancer Cell, 3, 363-375.
59. MacCarthy-Morrogh, L., Townsend, P.A., Purohit, A., Hejaz, H.A.M., Potter, B.V.L., Reed, M.J. & Packham, G. (2000). Differential effects of estrone and estrone-3-O-sulfamate derivatives on mitotic. Arrest, apoptosis, and microtubule assembly in human breast cancer cells. Cancer Res, 60, 5441-50.
60. Marsin, A.-S., Bertrand, L., Rider, M.H., Deprez, J., Beauloye, C., Vincent, M.-F., Van den Berghe, G., Carling, D. & Hue, L. (2000). Curr Biol, 10, 1247-1255.
61. Maschek, G., Savaraj, N., Priebe, W., Braunschweiger, P., Hamilton, K., Tidmarsh, G.F., De Young, L.R. & Lampidis, T.J. (2004). 2-deoxy-D-glucose increases the efficacy of adriamycin and paclitaxel in human osteosarcoma and non-small cell lung cancers in vivo. Cancer Research, 64, 31-4.
62. Moley, K.H. & Mueckler, M.M. (2000). Glucose transport and apoptosis. Apoptosis, 5, 99-105.
63. Mukhopadhyay, T. & Roth, J.A. (1997). Induction of apoptosis in human lung cancer cells after wild-type p53 activation by methoxyestradiol. Oncogene., 14, 379-384.
64. Nelson, C.A., Wang, J.Q., Leav, I. & Crane, P.D. (1996). The interaction among glucose transport, hexokinase, and glucose-6-phosphatase with respect to 3H-2-deoxyglucose retention in murine tumor models. Nucl Med Biol, 23, 533-41.
65. Newman, S.P., Leese, M.P., Purohit, A., James, D.R., Rennie, C.E., Potter, B.V. & Reed, M.J. (2004). Inhibition of in vitro angiogenesis by 2-methoxy- and 2-ethyl-estrogen sulfamates. International Journal of Cancer, 109, 533-40.
66. Noguchi, Y., Marat, D., Saito, A. & al, e. (1999). Expression of facilitative glucose transporters in gastric tumors. Hepatogastroenterology, 46, 2683-2689.
67. Pasqualini, J.R., Gelly, C., Nguyen, B.L. & Vella, C. (1989). Importance of estrogen sulfates in breast cancer. J Steroid Biochem, 34, 155-63.
68. Pelicano, H., Carney, D. & Huang, P. (2004). ROS stress in cancer cells and therapeutic implications. Drug Resist Updat, 7, 97-110.
69. Penta, J.S., Johnson, F.M., Wachsman, J.T. & Copeland, W.C. (2001). Mitochondrial DNA in human malignancy. Mutat Res, 488, 119-33.
70. Purohit, A., Froome, V.A., Wang, D.Y., Potter, B.V. & Reed, M.J. (1997). Measurement of oestrone sulphatase activity in white blood cells to monitor in vivo inhibition of steroid sulphatase activity by oestrone-3-O-sulphamate. J Steroid Biochem Mol Biol, 62, 45-51.
71. Purohit, A., Vernon, K.A., Hummelinck, A.E., Woo, L.W., Hejaz, H.A., Potter, B.V. & Reed, M.J. (1998). The development of A-ring modified analogues of oestrone-3-O-sulphamate as potent steroid sulphatase inhibitors with reduced oestrogenicity. J Steroid Biochem Mol Biol, 64, 269-75.
72. Purohit, A., Woo, L.W., Barrow, D., Hejaz, H.A., Nicholson, R.I., Potter, B.V. & Reed, M.J. (2001). Non-steroidal and steroidal sulfamates: new drugs for cancer therapy. Mol Cell Endocrinol, 171, 129-35.
73. Qin, S., Minami, Y., Hibi, M., Kurosaki, T. & Yamamura, H. (1997). Syk-dependent and -independent signaling cascades in B cells elicited by osmotic and oxidative stress. J Biol Chem, 272, 2098-103.
74. Raez, L.E., Rosenblatt, J., Schlesselman, J., Langmuir, V., Tidmarsh, G., Rocha-Lima, C., Papadopoulos, K., O'Connor, J., Baldie, P. & Lampidis, T. (2005). Combining glycolytic inhibitors with chemotherapy: Phase 1 trial of 2-deoxyglucose and docetaxel in patients with solid tumours. Journal of Clinical Oncology, 23, 3190.
75. Raobaikady, B., Purohit, A., Chander, S.K., Woo, L.W., Leese, M.P., Potter, B.V. & Reed, M.J. (2003). Inhibition of MCF-7 breast cancer cell proliferation and in vivo steroid sulphatase activity by 2-methoxyoestradiol-bis-sulphamate. J Steroid Biochem Mol Biol, 84, 351-8.
76. Raobaikady, B., Reed, M.J., Leese, M.P., Potter, B.V. & Purohit, A. (2005). Inhibition of MDA-MB-231 cell cycle progression and cell proliferation by C-2 substituted oestradiol mono- and bis-3-O-sulphamates. Int J Cancer, 117, 150-9.
77. Reed, M.J., Purohit, A., Woo, L.W.L. & Potter, B.V.L. (1996). The development of steroid sulphatase inhibitors. Endocr.-Re. Cancer, 3, 9-23.
78. Reiser, F., Way, D., Bemas, M., Witte, M. & Witte, C. (1998). Inhibition of normal and experimental angiotumour endothelial cell proliferation and cell cycle progression by 2-methoxyestradiol. Proc Soc Exp Biol Med, 219, 211-216.
79. Rivenzon-Segal, D., Rushkin, E., Polak-Charcon, S. & Degani, H. (2000). Glucose transporters and transport kinetics in retinoic acid-differentiated T47D human breast cancer cells. Am J Physiol Endocrinol Metab, 279, E508-19.
80. Rudlowski, C., Becker, A.J., Schroder, W. & al, e. (2003). GLUT1 mesenger RNA and protein induction relates to the malignant transformation of cervical cancer. Am J Clin Pathol, 120, 691-698.
81. Sattler, M., Quinnan, L.R., Pride, Y.B. & al, e. (2003). 2-Methoxyestradiol alters cell motility, migration, and adhesion. Blood, 102, 289-296.
82. Seegers, J.C., Aveling, M.L., Van Aswegen, C.H., Cross, M., Koch, F. & Joubert, W.S. (1989). The cytotoxic effects of estradiol-17 beta, catecholestradiols and methoxyestradiols on dividing MCF-7 and HeLa cells. J Steroid Biochem, 32, 797-809.
83. Seegers, J.C., Lottering, M.L., Grobler, C.J. & al, e. (1997). The mammalian metabolite, 2-methoxyestradiol, affects p53 levels and apoptosis induction in transformed cells but not in normal cells. J Steroid Bochem Mol Biol, 62, 253-267.
84. Suzuki, R.N., Newman, S.P., Purohit, A., Leese, M.P., Potter, B.V. & Reed, M.J. (2003a). Growth inhibition of multi-drug-resistant breast cancer cells by 2-methoxyoestradiol-bis-sulphamate and 2-ethyloestradiol-bis-sulphamate. Journal of Steroid Biochemistry & Molecular Biology, 84, 269-78.
85. Suzuki, T., Moriya, T., Ishida, T., Ohuchi, N. & Sasano, H. (2003b). Intracrine mechanism of estrogen synthesis in breast cancer. Biomed Pharmacother, 57, 460-2.
86. Van der Kaay, J., Beck, M., Gray, A. & Downes, C.P. (1999). Distinct phosphatidylinositol 3-kinase lipid products accumulate upon oxidative and osmotic stress and lead to different cellular responses. J Biol Chem, 274, 35963-8.
87. Vousden, K.H. & Lu, X. (2002). Live or let die: the cell's response to p53. Nature Reviews. Cancer, 2, 594-604.
88. Waki, A., Fujibayashi, Y., Yonekura, Y., Sadato, N., Ishii, Y. & Yokoyama, A. (1997). Reassessment of FDG uptake in tumor cells: high FDG uptake as a reflection of oxygen-independent glycolysis dominant energy production. Nucl Med Biol, 24, 665-70.
89. Waki, A., Kato, H., Yano, R., Sadato, N., Yokoyama, A., Ishii, Y., Yonekura, Y. & Fujibayashi, Y. (1998). The importance of glucose transport activity as the rate-limiting step of 2-deoxyglucose uptake in tumor cells in vitro. Nucl Med Biol, 25, 593-7.
90. Warburg, O.H. (1956). Science, 123, 309-314.
91. Waschsberger, P.R., Gressen, E.L., Bhala, A. & al, e. (2002). Variability in glucose transporter-1 levels and hexokinase activity in human melanoma. Melanoma Res, 12, 35-43.
92. Wood, L., Leese, M.P., Mouzakiti, A., Purohit, A., Potter, B.V., Reed, M.J. & Packham, G. (2004). 2-MeOE2bisMATE induces caspase-dependent apoptosis in CAL51 breast cancer cells and overcomes resistance to TRAIL via cooperative activation of caspases. Apoptosis, 9, 323-32.
93. Xiang, X., Saha, A.K., Wen, R., Ruderman, N.B. & Luo, Z. (2004). AMP-activated protein kinase activators can inhibit the growth of prostate cancer cells by multiple mechanisms. Biochem Biophys Res Commun, 321, 161-167.
94. Xu, R.-H., Pelicano, H., Zhou, Y., Carew, J.S., Feng, L., Bhalla, K.N., Keating, M.J. & Huang, P. (2005). Inhibition of Glycolysis in Cancer Cells: A Novel Strategy to Overcome Drug Resistance Associated with Mitochondrial Respiratory Defect and Hypoxia. Cancer Res, 65, 613-621.
95. Yue, T.L., Wang, X., Louden, C.S. & al, e. (1997). 2-Methoxyestradiol, an endogenous estrogen metabolite, induces apoptosis in endothelial cells and inhibits angiogenesis: Possible role for stress-activated protein kinase signaling pathway and Fas expression. Mol Pharmacol., 51, 951-962.
96. Zhou, Y., Hileman, E.O., Plunkett, W., Keating, M.J. & Huang, P. (2003). Free radical stress in chornic lymphocytic leukaemia cells and its role in cellular sensitivity to ROS-generating anticancer agents. Blood., 101, 4098-4104.
97. Zhu, Z., Jiang, W., McGinley, J.N. & Thompson, H.J. (2005). 2-Deoxyglucose as an Energy Restriction Mimetic Agent: EFfects on Mammary Carcinogenesis and on Mammary Tumor Cell Growth In vitro. Cancer Res, 65, 7023.
98. Zong, W.X., Edelstein, L.C., Chen, C., Bash, J. & Gelinas, C. (1999). The prosurvival Bcl-2 homolog Bfl-1/A1 is a direct transcriptional target of NF-kappaB that blocks TNFalpha-induced apoptosis. Genes & Development, 13, 382-7.

## Claims

1. A composition comprising
(a) 2-deoxy-D-glucose,
(b) a compound of the formula

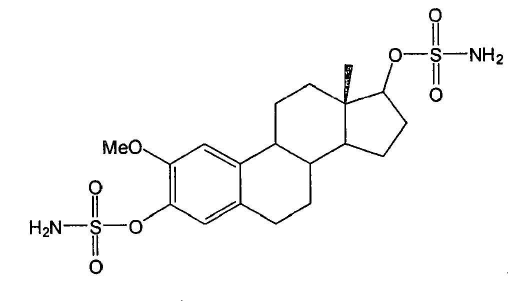

2. A product comprising
(a) 2-deoxy-D-glucose,
(b) a compound of the formula 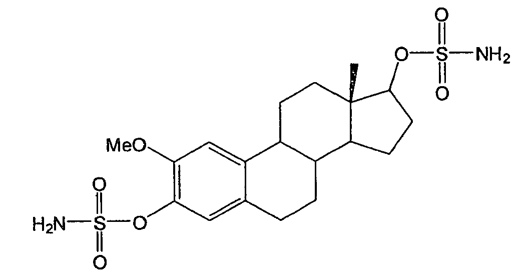 for simultaneous, separate or sequential use in the treatment of cancer.

3. A pharmaceutical composition comprising
(a) 2-deoxy-D-glucose,
(b) a compound of the formula 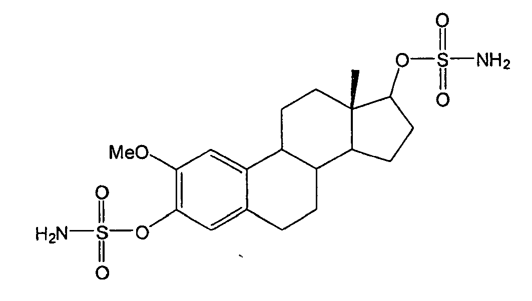 and
(c) a pharmaceutically acceptable carrier, diluent, excipient or adjuvant.

4. A composition comprising
(a) 2-deoxy-D-glucose,
(b) a compound of the formula 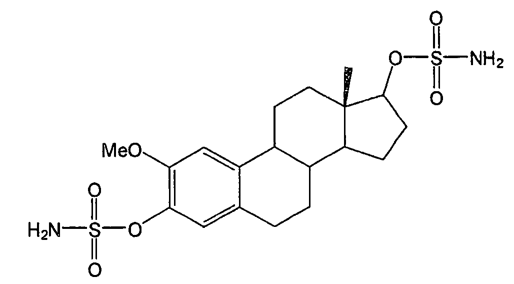 for use in medicine.

5. Use of a composition comprising
(a) 2-deoxy-D-glucose,
(b) a compound of the formula 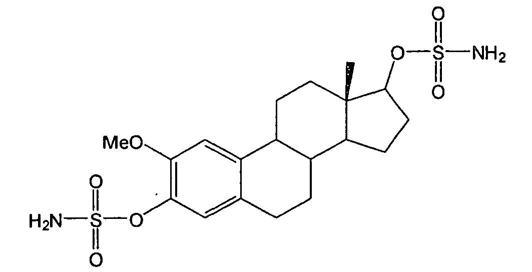 in the manufacture of a medicament for treating a cancer.

6. Use according to claim 5 wherein the cancer is breast cancer, ovarian cancer or prostate cancer.

7. A product comprising
(a) 2-deoxy-D-glucose,
(b) a compound of the formula 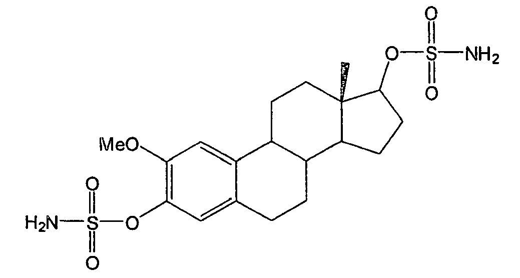 for use in the treatment of cancer,
wherein the 2-deoxy-D-glucose and the compound (b) are administered sequentially.

8. Use of a composition comprising
(a) 2-deoxy-D-glucose,
(b) a compound of the formula 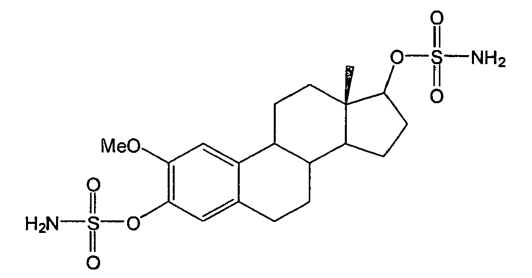 in the manufacture of a medicament for decreasing ATP levels in a tumour.

## Patentansprüche

1. Eine Zusammensetzung, umfassend
(a) 2-Desoxy-D-glucose,
(b) eine Verbindung der Formel

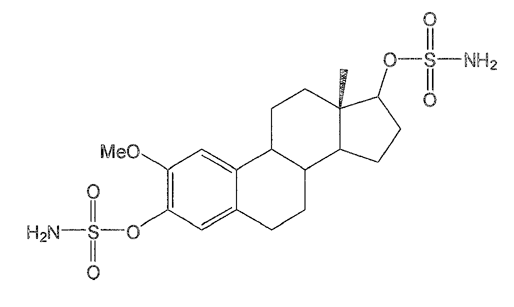

2. Ein Produkt, umfassend
(a) 2-Desoxy-D-glucose,
(b) eine Verbindung der Formel 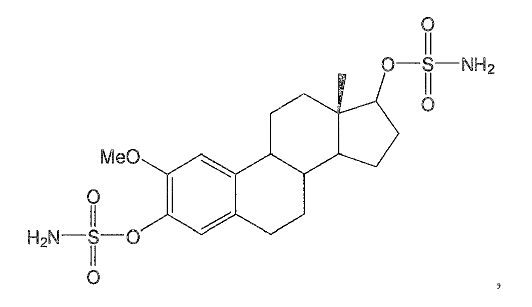 zur gleichzeitigen, separaten oder sequentiellen Verwerdung bei der Behandlung von Krebs.

3. Eine pharmazeutische Zusammensetzung, umfassend
(a) 2-Desoxy-D-glucose,
(b) eine Verbindung der Formel 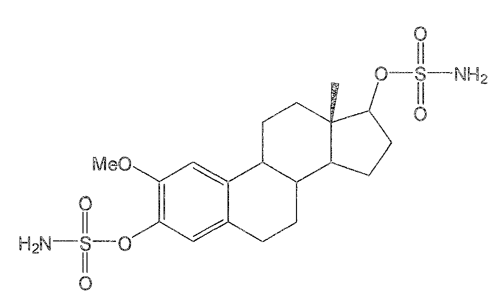 und
(c) einen pharmazeutisch annehmbaren Träger, ein pharmazeutisch annehmbares Verdünnungsmittel, ein pharmazeutisch annehmbares Vehikel oder einen pharmazeutisch annehmbaren Hilfsstoff.

4. Eine Zusammensetzung, umfassend
(a) 2-Desoxy-D-glucose,
(b) eine Verbindung der Formel 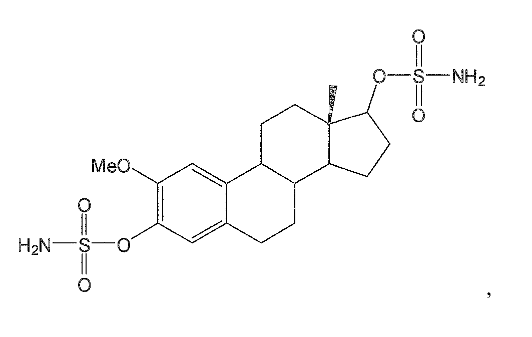 zur Verwerdung in der Medizin.

5. Verwendung einer Zusammensetzung, umfassend
(a) 2-Desoxy-D-glucose,
(b) eine Verbindung der Formel 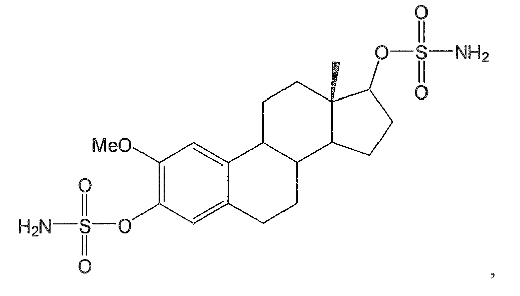 bei der Herstellung eines Medikaments zur Behandlung von Krebs.

6. Verwerdung gemäß Anspruch 5, wobei der krebs Brustkrebs, Ovarialkarzonom oder Prostatakrebs ist.

7. Ein Produkt, umfassend
(a) 2-Desoxy-D-glucose,
(b) eine Verbindung der Formel 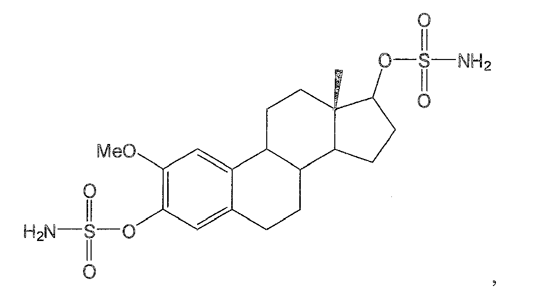 zur Verwerdung bei der Behandlung von Krebs,
wobei die 2-Desoxy-D-glucose und die Verbindung (b) der Reihe nach verabreicht werden.

8. Verwerdung einer Zusammensetzung, umfassend
(a) 2-Desoxy-D-glucose,
(b) eine Verbindung der Formel 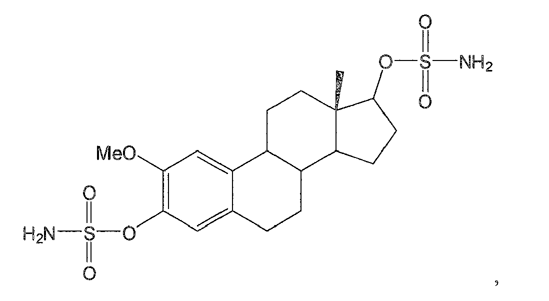 bei der Herstellung eines Medikaments zur Verringerung des ATP-Gehalts in einem Tumor.

## Revendications

1. Une composition comprenant
(a) 2-desoxy-D-glucose,
(b) une composé de formule

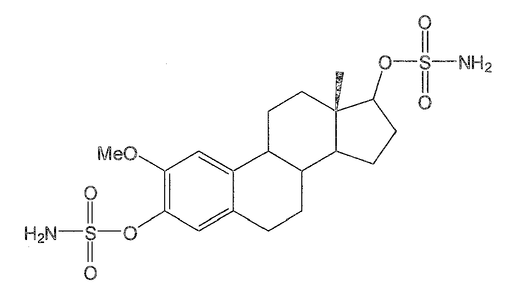

2. Un produit comprenant
(a) 2-desoxy-D-glucose,
(b) un composé de formule 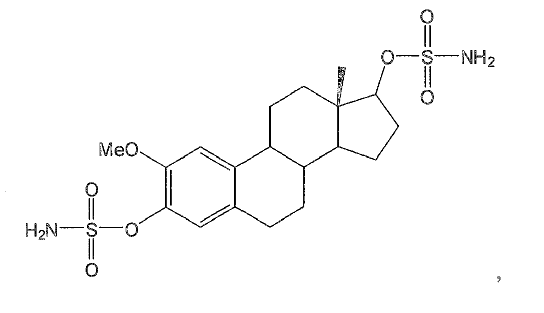 pour une administration simultanée, séparée ou séquencée dans le traitement du cancer.

3. Une composition pharmaceutique comprenant
(a) 2-desoxy-D-glucose,
(b) un composé de formule 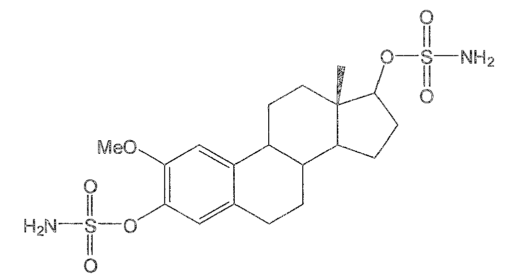 et
(c) un support, diluant, excipient ou adjuvant pharmaceutiquement acceptable.

4. Une composition comprenant
(a) 2-desoxy-D-glucose,
(b) un composition de formule 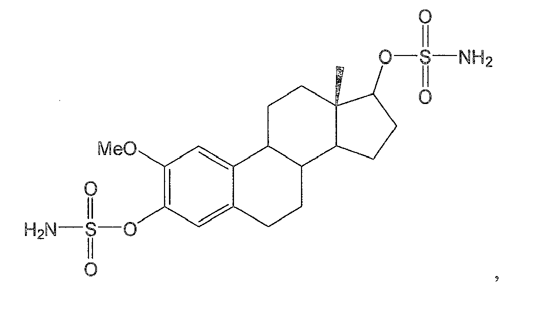 pour une utilisation en médecine.

5. Utilisation d'une composition comprenant
(a) 2-desoxy-D-glucose,
(b) un composé de formule 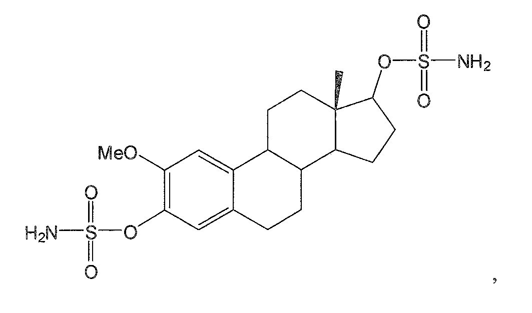 dans la préparation d'un médicament pour le traitement du cancer.

6. Utilisation selon la revendication 5 dans laquelle le cancer est le cancer du sein, des ovaires ou de la prostate.

7. Un produit comprenant
(a) 2-desoxy-D-glucose,
(b) un composé de formule 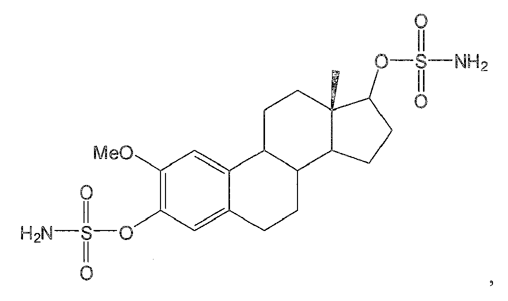 pour une utilisation dans le traitement du cancer,
dans lequel le 2-desoxy-D-glucose et le compound (b) sont administrés de façon séquencée.

8. Utilisation d'une composition comprenant
(a) 2-desoxy-D-glucose,
(b) un composé de formule 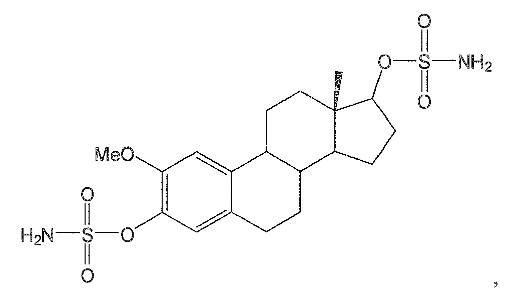 dans la prépartion d'un médicament qui décroit les niveaux d'ATP dans une tumeur.
